# EUROPEAN PATENT APPLICATION

(11) **EP 4 768 062 A1**
(43) Date of publication of application: **01.07.2026**
(21) Application number: 25223167.5
(22) Date of filing: 12.12.2025
(51) Int. Cl.: A61M 25/00, A61B 17/3205

(54) **MEDICAL CATHETER, SURGICAL INSTRUMENT, AND MEDICAL CATHETER MANUFACTURING METHOD**

(30) Priority: 27.12.2024 CN 202411946815
(71) Applicant: Micro-Tech (Nanjing) Co., Ltd., Nanjing, Jiangsu 210032 (CN)
(72) Inventor: XIE, Huan, NANJING, 210032 (CN); TANG, Zhi, NANJING, 210032 (CN); ZHOU, Yunfeng, NANJING, 210032 (CN); WANG, Qi, NANJING, 210032 (CN)
(74) Representative: Locas, Davide

(57) **Abstract**

The present invention provides a medical catheter, a surgical instrument, and a medical catheter manufacturing method, which relates to the technical field of medical device. The medical catheter provided by the present invention adopts a structure in which a support tube is installed inside an outer tube body and the support tube extends along the inner wall of the outer tube body. This structure not only allows the medical catheter to bend and easily pass through curved channels, but also maintains good structural strength and support when the outer tube body has a small diameter and thin wall. It is particularly suitable for surgical instruments that need to be inserted through narrow channels.

## Description

### CROSS-REFERENCE TO RELATED APPLICATIONS

The present application claims priority to Chinese Patent Application No. 202411946815.9 filed on December 27, 2024 with the Chinese Patent Office, and entitled "MEDICAL CATHETER, SURGICAL INSTRUMENT, AND MEDICAL CATHETER MANUFACTURING METHOD", the entire contents of which are incorporated herein by reference.

### TECHNICAL FIELD

The present invention belongs to the technical field of medical device, particularly to a medical catheter, a surgical instrument, and a medical catheter manufacturing method.

### BACKGROUND ART

Surgical instruments such as snare devices usually use medical catheters to transport the working portion to a specific location inside the patient's body under endoscopy. However, for endoscopes with narrow working channel, such as choledochoscopes, common medical catheters cannot be inserted through the channel. In order to meet the requirements of small channel insertion, it is usually necessary to reduce the diameter and wall thickness of the medical catheter, which will lead to a decrease in the support of the medical catheter, thereby increasing the risk of deformation or damage to the medical catheter and easily affecting the smooth progress of the surgery.

### SUMMARY

The purpose of the present invention is to provide a medical catheter, a surgical instrument, and a medical catheter processing method to alleviate the technical problem of weak support for small-diameter medical catheters.

In a first aspect, the present invention provides a medical catheter comprising: an outer tube body and a support tube installed inside the outer tube body;
the support tube extends along an inner wall of the outer tube body.

Based on the first aspect, the present invention provides a first possible embodiment of the first aspect, wherein the support tube comprises a mesh structure or spiral structure adhered to the inner wall of the outer tube body.

Based on the first possible embodiment of the first aspect, the present invention provides a second possible embodiment of the first aspect, wherein the support tube comprises a fixed portion fixed relative to the outer tube body, and a movable portion movable relative to the outer tube body;
the movable portion moves relative to the fixed portion to change a pitch of the support tube or a mesh size of the support tube.

Based on the first possible embodiment of the first aspect, the present invention provides a third possible embodiment of the first aspect, wherein a mesh density of the support tube gradually increases from a proximal end to a distal end, or a pitch of the support tube gradually decreases from a proximal end to a distal end.

Based on the first possible embodiment of the first aspect, the present invention provides a fourth possible embodiment of the first aspect, wherein a mesh density at a distal end of the support tube is greater than that at a proximal end of the support tube, or the pitch at a distal end of the support tube is smaller than that at a proximal end of the support tube.

Based on the first aspect, the present invention provides a fifth possible embodiment of the first aspect, wherein the support tube is formed by winding a metal wire, or the support tube is formed by carving a metal tube.

Based on the first aspect, the present invention provides a sixth possible embodiment of the first aspect, wherein a hardness of a distal end of the support tube is smaller than that of a proximal end the support tube.

Based on the first aspect, the present invention provides a seventh possible embodiment of the first aspect, wherein a wall thickness of a distal end of the support tube is smaller than that of a proximal end of the support tube.

Based on the first aspect, the present invention provides an eighth embodiment of the first aspect, wherein a distal end of the support tube is spaced apart from a distal end of the outer tube body in a direction from the proximal end to the distal end, to make the support tube apart from the distal end of the outer tube body to form an instrument accommodation space.

In a second aspect, the present invention provides a surgical instrument comprising a working portion, a transmission portion, a handle, and the medical catheter according to the first aspect;
the working portion is slidably inserted in the outer tube body, and the transmission portion passes through the medical catheter, with a distal end of the transmission portion connected to the working portion and a proximal end the transmission portion connected to the handle.

Based on the second aspect, the present invention provides a first possible embodiment of the second aspect, wherein the handle comprises a core rod and a slider that slidably mates the core rod;
a proximal end of the outer tube body is connected to the core rod, and a proximal end of the transmission portion is connected to the slider.

Based on the first possible embodiment of the second aspect, the present invention provides a second possible embodiment of the second aspect, wherein the transmission portion comprises a connecting tube and a cable;
the connecting tube slides and fits inside the outer tube body, and the connecting tube is connected to the working portion; a distal end of cable is connected to connecting tube, and a proximal end of cable is connected to slider.

Based on the second possible embodiment of the second aspect, the present invention provides a third possible embodiment of the second aspect, wherein a surface of the cable is coated with a lubricating coating.

Based on the second aspect, the present invention provides a fourth possible embodiment of the second aspect, wherein the working portion comprises a snare; the snare has an elastic tendency to expand radially.

In a third aspect, the present invention provides a medical catheter manufacturing method comprising following steps:
installing a support tube inside an outer tube body;
wherein the support tube has a mesh structure or spiral structure extending along an inner wall of the outer tube body.

Based on the third aspect, the support tube has a fixed portion that is fixed relative to the outer tube body, and a movable portion that is movable relative to the support tube;
the medical catheter manufacturing method further comprises: adjusting the movement of the movable portion relative to the fixed portion to change the mesh size or pitch of the support tube.

The embodiments of the present invention bring the following beneficial effects: adopting the method of installing a support tube inside the outer tube body and extending the support tube along the inner wall of the outer tube body not only enables the medical catheter to bend and easily pass through the curved channel, but also maintains better support in the case of a thin diameter and wall thickness of the outer tube body, especially suitable for surgical instruments inserted through narrow channels.

In order to make the above objectives, features, and advantages of the present invention more obvious and understandable, the following preferred embodiments are presented in detail with the accompanying drawings.

### BRIEF DESCRIPTION OF THE DRAWINGS

In order to provide a clearer explanation of the specific embodiments of the present invention or the technical solutions in related technologies, a brief introduction will be given to the accompanying drawings required for the description of the specific embodiments or related technologies. It is obvious that the accompanying drawings described below are some embodiments of the present invention. For those skilled in the art, other drawings can be obtained based on these drawings without creative labor.
Figure 1 shows a schematic diagram of a surgical instrument provided by an embodiment of the present invention in the state of releasing the working portion;
Figure 2 shows a schematic diagram of a surgical instrument provided by an embodiment of the present invention in the state of retracting the working portion;
Figure 3 shows a schematic diagram of a first kind of support tube of a medical catheter provided by an embodiment of the present invention;
Figure 4 shows a schematic diagram of a second kind of support tube of a medical catheter provided by an embodiment of the present invention;
Figure 5 shows a schematic diagram of a third kind of support tube of a medical catheter provided by an embodiment of the present invention;
Figure 6 shows a schematic diagram of a fourth kind of support tube of a medical catheter provided by an embodiment of the present invention;
Figure 7 shows a schematic diagram of a fifth kind of support tube of a medical catheter provided by an embodiment of the present invention;
Figure 8 shows a schematic diagram of a sixth kind of support tube of a medical catheter provided by an embodiment of the present invention;
Figure 9 shows a schematic diagram of a seventh kind of support tube of a medical catheter provided by an embodiment of the present invention;
Figure 10 shows a schematic diagram of an eighth kind of support tube of a medical catheter provided by an embodiment of the present invention in a kind of state;
Figure 11 shows a schematic diagram of an eighth kind of support tube of a medical catheter provided by an embodiment of the present invention in another kind of state;
Figure 12 shows a schematic diagram of a nineth kind of support tube of a medical catheter provided by an embodiment of the present invention in a kind of state;
Figure 13 shows a schematic diagram of a nineth kind of support tube of a medical catheter provided by an embodiment of the present invention in another kind of state;
Figure 14 shows a schematic diagram of the opened state of jaws provided by an embodiment of the present invention;
Figure 15 shows a schematic diagram of the closed state of jaws provided by an embodiment of the present invention;
Figure 16 shows a schematic diagram of the opened state of a clip provided by an embodiment of the present invention;
Figure 17 shows a schematic diagram of the closed state of a clip provided by an embodiment of the present invention;
Figure 18 shows a schematic diagram of the extended state of a needle provided by an embodiment of the present invention;
Figure 19 shows a schematic diagram of the retracted state of a needle provided by an embodiment of the present invention;
Figure 20 shows a schematic diagram of the extended state of a knife provided by an embodiment of the present invention;
Figure 21 shows a schematic diagram of the retracted state of a knife provided by an embodiment of the present invention.

Icons: 100- outer tube body; 101- instrument accommodation space; 200- support tube; 201- fixed portion; 202- movable portion; 300- working portion; 301- jaws; 302- clip; 303-needle; 304- knife; 400- transmission portion; 410- connecting tube; 420- cable; 500- handle; 510- core rod; 520- slider; 521- conductive plug; 522- injection port.

### DETAILED DESCRIPTION OF EMBODIMENTS

The following will provide a clear and complete description of the technical solution of the present invention with reference to the accompanying drawings. Obviously, the described embodiments are only a part of the embodiments of the present invention, not all of them. Based on the embodiments of the present invention, all other embodiments obtained by ordinary skilled persons in the art without creative labor are within the scope of protection of the present invention.

In the description of the present invention, it should be noted that the terms "center", "up", "down", "left", "right", "vertical", "horizontal", "inside", "outside" and other directional or positional relationships indicated are based on the directional or positional relationships shown in the accompanying drawings, only for the convenience of describing the present invention and simplifying the description, and do not indicate or imply that the device or element referred to must have a specific orientation, be constructed and operated in a specific orientation, and therefore cannot be understood as a limitation of the present invention. In addition, "near end" and "far end" refer to the operator or operating handle as a reference to obtain the corresponding orientation reference. The terms "first", "second", and "third" are only used to describe name differences and should not be understood as indicating or implying relative importance. Unless otherwise specified, all physical quantities in the equations are to be understood as the base quantities of the International System of Units, or as derived quantities derived from base quantities through mathematical operations such as multiplication, division, differentiation, or integration.

In the description of the present invention, it should be noted that unless otherwise specified and limited, the terms "installation", " connected", and "connection" should be broadly understood, for example, they can be fixed connections, detachable connections, or integral connections; they can be a mechanical connection or an electrical connection; they can be directly connected, indirectly connected through an intermediate medium, or connected internally between two components. For ordinary technical personnel in this field, the specific meanings of the above terms in the present invention can be understood in specific situations.

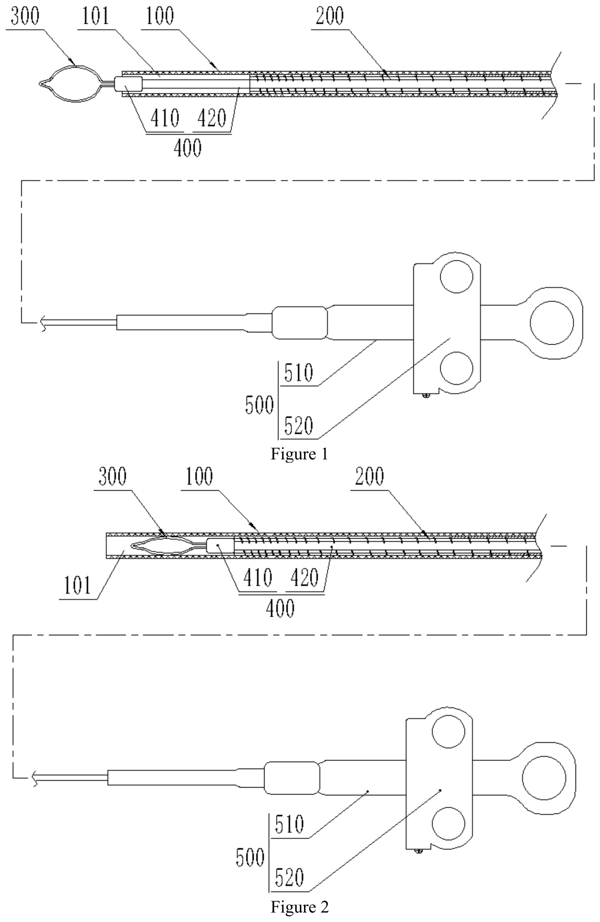

As shown in Figures 1, 2, 3, 4, and 5, the medical catheter provided by the embodiment of the present invention includes: an outer tube body 100 and a support tube 200 installed inside the outer tube body 100; the support tube 200 extends along the inner wall of the outer tube body 100.

Specifically, the outer tube body 100 is made of polymeric_materials such as polytetrafluoroethylene (PTFE) or polytetrafluoroethylene propylene (FEP), which have temperature and voltage resistance properties. With the reduction of the outer diameter and wall thickness of the outer tube body 100, a support tube 200 is installed inside the outer tube body 100 to achieve structural reinforcement. The support tube 200 can be made of stainless steel, nickel titanium alloy, or other equivalent high hardness materials, which can significantly improve the strength and support of medical catheters.

Furthermore, the support tube 200 includes a mesh structure or spiral structure adhered to the inner wall of the outer tube body 100. The inner or outer surface of the support tube 200 can be coated with polymer material, that is, the inner or outer surface of the support tube 200 can be covered with a film.

In alternative embodiments, the support tube 200 can be processed into a mesh structure with a specific mesh density, or into a helical structure with a specific pitch.

It should be noted that the mesh density of the support tube 200 mainly refers to the number of mesh holes within a certain size range in the axial direction of the support tube 200. As the mesh density increases, the number of mesh holes within a certain size range in the axial direction of the support tube 200 also increases.

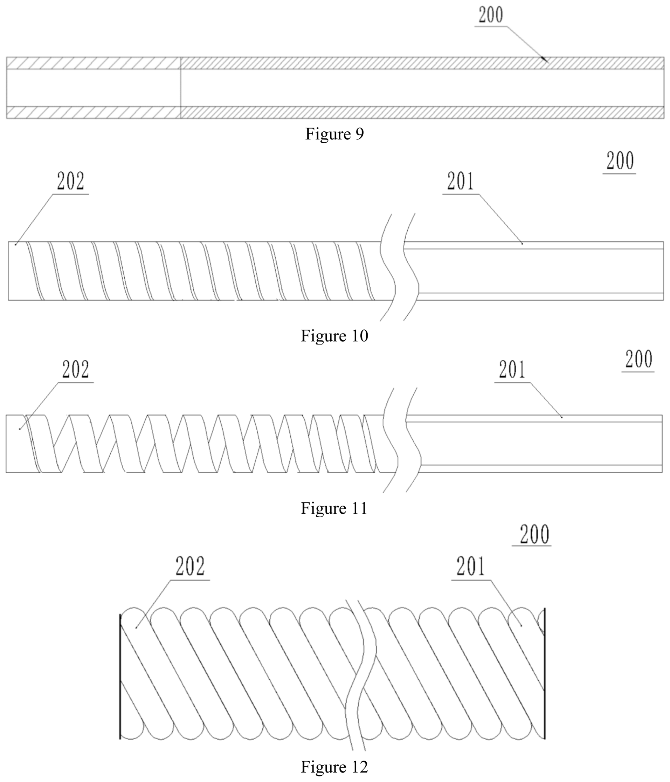

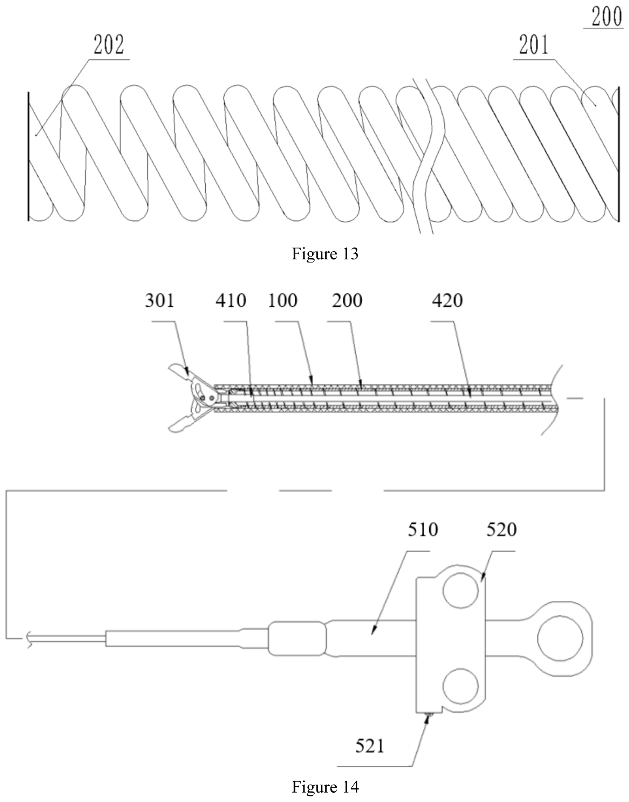

As shown in Figures 10, 11, 12, and 13, in the embodiment of the present invention, the support tube 200 has a fixed portion 201 fixed relative to the outer tube body 100, and a movable portion 202 movable relative to the outer tube body 100; the movable portion 202 moves relative to the fixed portion 201 to change the pitch of the support tube 200 or the mesh size in the axial direction of the support tube 200. By changing the mesh size of the mesh structure or the pitch of the spiral structure, the flexibility and support of the support tube 200 can be adjusted. In other words, by increasing the mesh size or pitch in the axial direction of the support tube 200, the hollow structure of the support tube 200 within a certain length range can be increased, making the support tube 200 easier to bend and making medical catheters more suitable for insertion through curved endoscope working channel. Referring to Figures 10 and 12, in the initial state, the spiral structure of the support tube 200 maintains a shape with a certain pitch density. When the movable portion 202 moves away from the fixed portion 201 and pulls the support tube 200 to elongate, as shown in Figures 11 and 13, the gap and pitch of the spiral structure increase, making the support tube 200 more prone to bending.

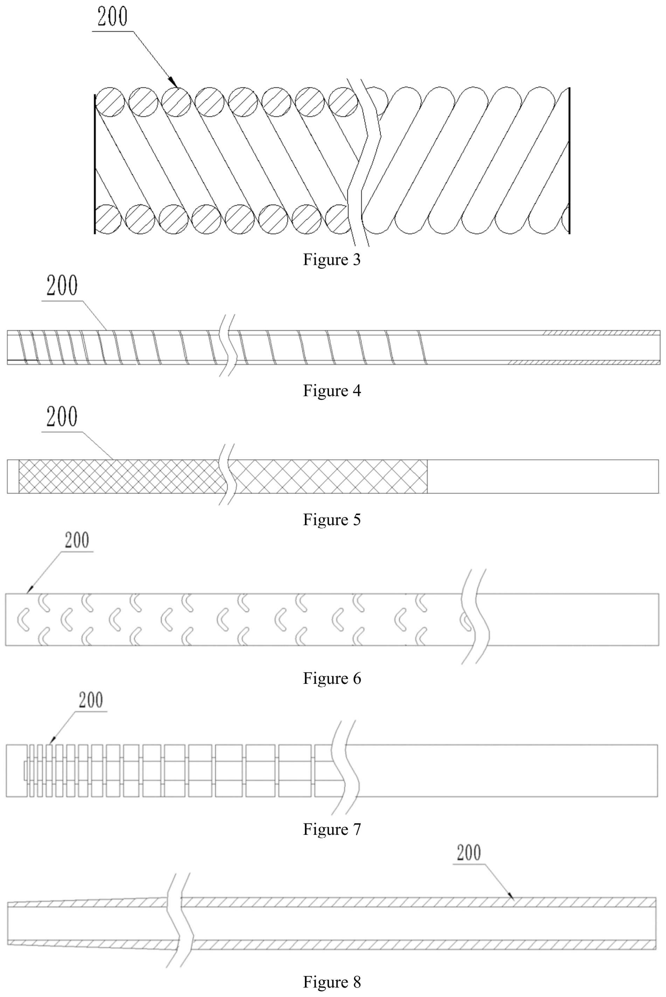

As shown in Figures 4, 5, 6, and 7, in alternative embodiments, the mesh density of the support tube 200 gradually increases from the proximal end to the distal end, or the pitch of the support tube 200 gradually decreases from the proximal end to the distal end. The distal mesh of the support tube 200 is dense, or the pitch of the distal end of the support tube 200 is smaller and the hollow portion of the spiral structure is more compact, making the distal end of the support tube 200 easy to bend and over bend.

In alternative embodiments, the mesh density at the distal end of the support tube 200 is greater than that at the proximal end, or the pitch at the distal end of the support tube 200 is smaller than that at the proximal end. The mesh size or pitch of the distal end of the support tube 200 along its own axis is greater than or equal to 0.03mm, and the mesh size or pitch of the proximal end of the support tube 200 along its own axis is greater than or equal to 0.3mm. Among them, a smaller pitch or mesh structure is used within the range of 1mm to 100mm to the distal end of the support tube 200, or within the range of 1mm to 500mm. At the distal end, the pitch is smaller or the mesh density is higher, the pore distribution is dense, and it is easier to bend and pass through an endoscopic bend; the proximal pitch is larger or the mesh density is smaller, the pore distribution is sparse, the support performance is better, and it is easy to push.

As shown in Figure 9, in an alternative embodiment, the hardness of the distal end of the support tube 200 can be made smaller than that of the proximal end, which can also improve the bending performance of the distal end of the support tube 200.

As shown in Figure 8, in an alternative embodiment, the wall thickness of the distal end of the support tube 200 is smaller than that of the proximal end, and the wall thickness of the distal end of the support tube 200 is small, making it easier to bend; the proximal wall thickness of support tube 200 is larger, providing better support.

Referring to Figure 3, in one alternative embodiment, the support tube 200 can be formed by winding metal wires.

Referring to Figures 4 and 5, in another alternative embodiment, the support tube 200 can be formed by carving metal tubes, which can be spiral carved or carved with mesh holes along the side walls of the metal tubes to form a spiral or mesh structure.

As shown in Figures 1 and 2, the distal end of the support tube 200 is spaced apart from the distal end of the outer tube body 100 in the direction from the proximal end to the distal end, thereby avoiding that the support tube 200 extends to the distal end of the outer tube body 100. The support tube 200 is set apart from the distal end of the outer tube body 100 to form an instrument accommodation space 101, on the one hand, leaving enough space for the instrument accommodation space 101, and on the other hand, reducing the resistance released by the working portion 300 towards the distal end, and making it easier for the working portion 300 to get back into the instrument accommodation space 101. The working portion 300 can be connected to the power supply through a conductor medium.

As shown in Figure 1 and Figure 2, the surgical instrument provided by the embodiment of the present invention includes: a working portion 300, a transmission portion 400, a handle 500, and the medical catheter described in the above embodiment; the working portion 300 is slidably inserted in the outer tube body 100, and the transmission portion 400 passes through the medical catheter, with the distal end of the transmission portion 400 connected to the working portion 300 and the proximal end connected to the handle 500. The surgical instrument may be an electrosurgical instrument.

The handle 500 transmits pushing force and pulling force through the transmission portion 400, which can push the working portion 300 to release from the distal end of the outer tube body 100, or pull the working portion 300 to get back into the interior of the outer tube body 100 from the distal end of the outer tube body 100.

In an optional implementation, the handle 500 includes a core rod 510 and a slider 520 that slidably mates the core rod 510; the proximal end of the outer tube body 100 is connected to the core rod 510, and the proximal end of the transmission portion 400 is connected to the slider 520. The proximal end of the outer tube body 100 is supported by the core rod 510. By operating the slider 520 to slide relative to the core rod 510, the transmission portion 400 can be pushed and pulled, thereby achieving the release and retraction of the working portion 300.

In an optional implementation, the transmission portion 400 includes: a connecting tube 410 and a cable 420; the connecting tube 410 slides and fits inside the outer tube body 100, and the connecting tube 410 is connected to the working portion 300; the distal end of cable 420 is connected to connecting tube 410, and the proximal end of cable 420 is connected to slider 520. The connection between the connecting tube 410 and the working portion 300, as well as the connection between the connecting tube 410 and the cable 420, can be welded or riveted. The cable 420 is fitted with a gap inside the medical catheter. By pushing or pulling the cable 420 through the slider 520, pushing force or pulling force can be transmitted, thereby achieving the release and retraction of the working portion 300.

Furthermore, the cable 420 is made of materials such as stainless steel and nickel titanium, and can be configured as a single wire structure or a cable structure, with flexibility. The surface of the cable 420 is coated with a lubricating coating, which can reduce the frictional resistance of the cable 420 inside the medical catheter, making the release and retraction operations of the working portion 300 more convenient.

Furthermore, the working portion 300 may include a snare as shown in Figure 1, which has an elastic tendency to expand radially. Among them, the ring can be made of materials such as stainless steel, nickel titanium, etc., and can also be made of a mixed structure of multiple materials. In the unfolded state, it can be configured as a diamond shaped structure, elliptical structure, hexagonal structure, etc.

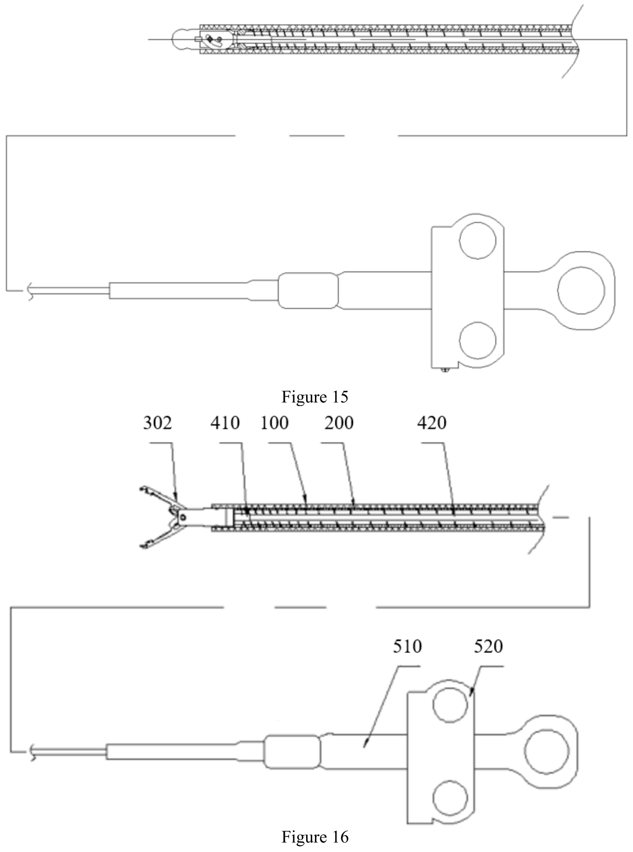

As shown in Figures 14 and 15, the working portion 300 may include jaws 301, which can be opened and closed radially. As shown in Figure 14, in the open state, the jaws 301 extends out of the outer tube body 100 for sampling, such as grasping tissue, and can also be used for hemostasis. As shown in Figure 15, in the closed state, the jaws 301 can be retracted into the outer tube body 100, which can prevent thermal damage to unexpected portions when high-frequency electricity is connected. The slider can be equipped with a conductive plug 521 to provide power to the jaws 301.

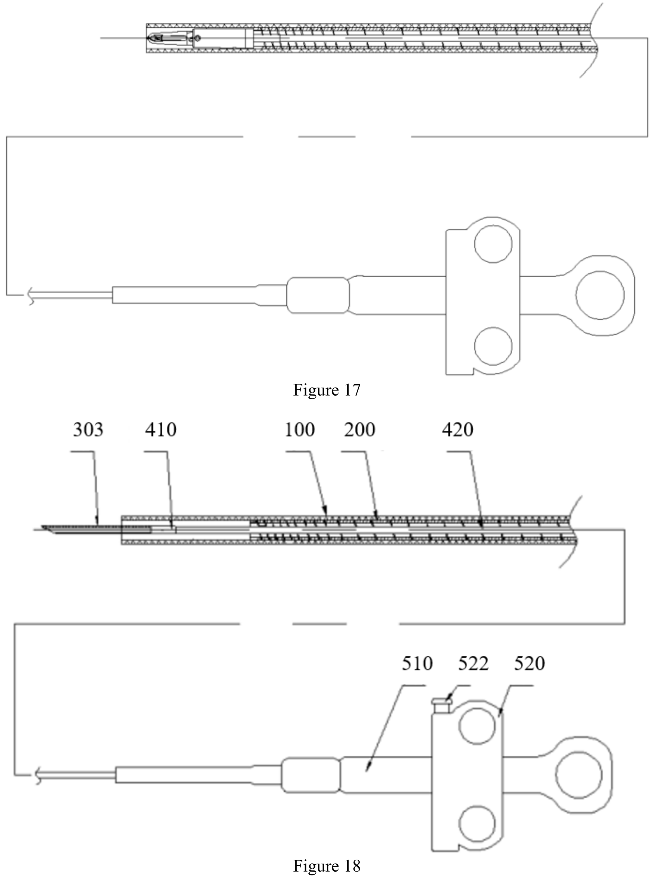

As shown in Figures 16 and 17, the working portion 300 may include a clip 302, which can be opened and closed radially. As shown in Figure 16, in the open state, the clip 302 extends out of the outer tube body 100, it can be used to clamp tissue etc., and it can be used for hemostasis. As shown in Figure 17, in the closed state, the clip 302 can be retracted into the outer tube body 100, preventing the teeth of the clip 302 from damaging the endoscope working channel when passing through it.

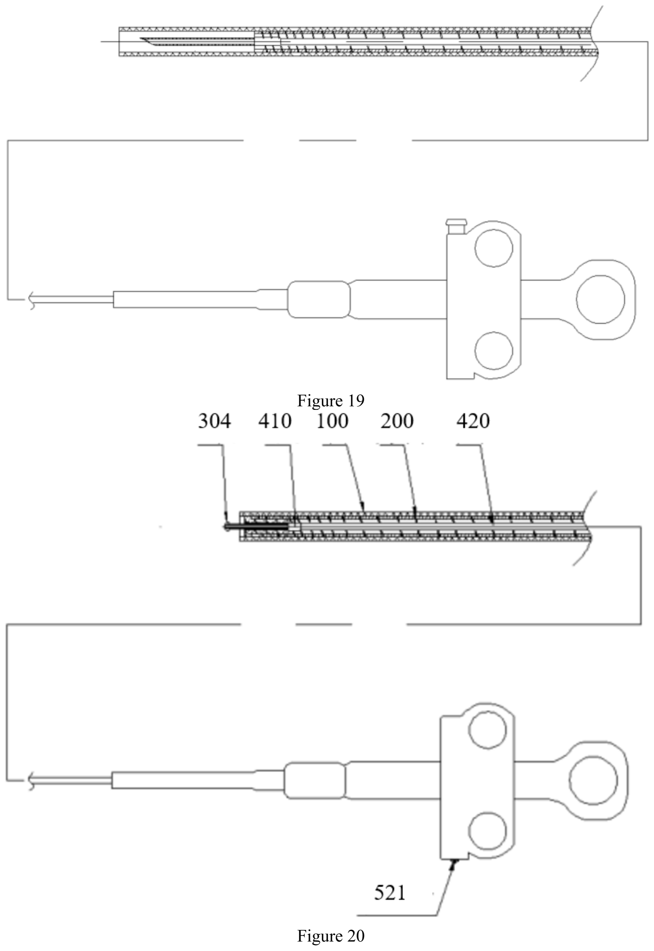

As shown in Figures 18 and 19, the working portion 300 may include a needle 303, which can be extended or retracted. The cable 420 may be of hollow construction, and the cable 420 is connected to needle 303 through connecting tube 410 or directly connected to needle 303. As shown in Figure 18, when the needle 303 extends out of the outer tube body 100, the injection function can be achieved through the injection port 522. As shown in Figure 19, when the needle 303 is retracted, the space between the distal end of the outer tube body 100 and the distal end of the support tube 200 increases the gap between the needle tip of the needle 303 and the outer tube body 100, reducing the risk of the needle tip of the needle 303 puncturing the outer tube body 100.

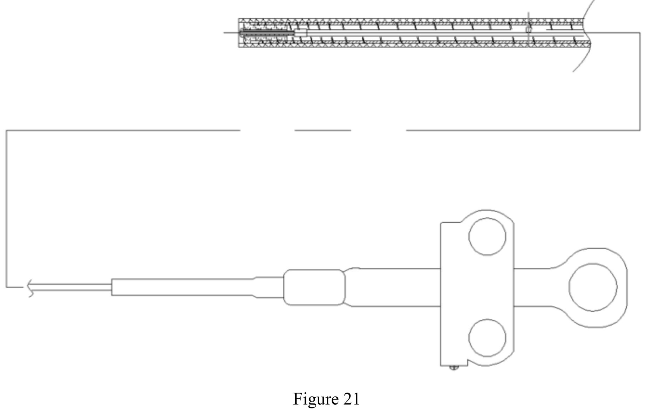

As shown in Figures 20 and 21, the working portion 300 may include a knife 304, which can be extended or retracted. The cable 420 may be of hollow construction, and be connected to knife 304 through connecting tube 410. As shown in Figure 20, when the knife 304 extends out of the outer tube body 100, functions such as marking, cutting (by connecting to electricity), and injection can be achieved. As shown in Figure 21, when the knife 304 cannot be fully retracted into the support tube 200, the distal end of the outer tube body 100 can accommodate the tip of the knife 304, reducing the risk of unexpected thermal damage.

The medical catheter manufacturing method provided in the embodiments of the present invention includes the following steps:

Installing a support tube 200 inside an outer tube body 100;

Wherein the support tube 200 has a mesh structure or spiral structure extending along the inner wall of the outer tube body 100.

The medical catheter obtained by this manufacturing method has improved structural strength and support by the support tube 200, while the outer diameter of the outer tube body100 is reduced and the wall thickness is reduced. It can be bent and inserted along the curved endoscope working channel, especially suitable for use in narrow endoscope working channels.

In an optional implementation, the support tube 200 can have a fixed portion 201 that is fixed relative to the outer tube body 100, and a movable portion 202 that is movable relative to the support tube 200; the medical catheter manufacturing method further includes adjusting the movement of the movable portion 202 relative to the fixed portion 201 to change the mesh size or pitch of the support tube 200. By moving the movable portion 202 relative to the fixed portion 201 along the axial direction of the support tube 200, the mesh size or pitch in the axial direction of the support tube 200 changes accordingly, thereby changing the bending resistance and support performance of the support tube 200. Therefore, the flexibility and support of the support tube 200 can be adjusted as needed. When the degree of bending of the endoscope working channel is large, the mesh size or pitch in the axial direction of the support tube 200 can be increased to make the medical catheter easier to bend, thereby making the insertion of surgical instruments smoother.

Finally, it should be noted that the above embodiments are only used to illustrate the technical solution of the present invention, and not to limit it; although the present invention has been described in detail with reference to the aforementioned embodiments, those skilled in the art should understand that they can still modify the technical solutions described in the aforementioned embodiments, or equivalently replace some or all of the technical features; these modifications or substitutions do not deviate from the essence of the corresponding technical solutions from the scope of the various embodiments of the present invention.

## Claims

1. A medical catheter **characterised in that** the medical catheter comprises: an outer tube body (100) and a support tube (200) installed inside the outer tube body (100);
the support tube (200) extends along an inner wall of the outer tube body (100);
optionally, a surface of the support tube (200) is covered with a polymer material.

2. The medical catheter according to claim 1, wherein the support tube (200) comprises a mesh structure or spiral structure adhered to the inner wall of the outer tube body (100).

3. The medical catheter according to claim 2, wherein the support tube (200) comprises a fixed portion (201) fixed relative to the outer tube body (100), and a movable portion (202) movable relative to the outer tube body (100);
the movable portion (202) moves relative to the fixed portion (201) to change a pitch of the support tube (200) or a mesh size of the support tube (200).

4. The medical catheter according to claim 2, wherein a mesh density of the support tube (200) gradually increases from a proximal end to a distal end, or a pitch of the support tube (200) gradually decreases from a proximal end to a distal end.

5. The medical catheter according to claim 2, wherein a mesh density at a distal end of the support tube (200) is greater than that at a proximal end of the support tube (200), or the pitch at a distal end of the support tube (200) is smaller than that at a proximal end of the support tube (200).

6. The medical catheter according to claim 1 or 2, wherein the support tube (200) is formed by winding a metal wire, or the support tube (200) is formed by carving a metal tube.

7. The medical catheter according to claim 1, wherein a hardness of a distal end of the support tube (200) is smaller than that of a proximal end the support tube (200).

8. The medical catheter according to claim 1, wherein a wall thickness of a distal end of the support tube (200) is smaller than that of a proximal end of the support tube (200).

9. The medical catheter according to claim 1, wherein a distal end of the support tube (200) is spaced apart from a distal end of the outer tube body (100) in a direction from the proximal end to the distal end, to make the support tube (200) apart from the distal end of the outer tube body (100) to form an instrument accommodation space (101).

10. A surgical instrument **characterised in that** the surgical instrument comprises a working portion (300), a transmission portion (400), a handle (500), and the medical catheter according to any one of claims 1-9;
the working portion (300) is slidably inserted in the outer tube body (100), and the transmission portion (400) passes through the medical catheter, with a distal end of the transmission portion (400) connected to the working portion (300) and a proximal end the transmission portion (400) connected to the handle (500);
optionally, the surgical instrument is an electrical surgical instrument;
optionally, the working portion (300) comprises at least one of the following: jaws (301), a clip (302), a needle (303), or a knife (304).

11. The surgical instrument according to claim 10, wherein the handle (500) comprises a core rod (510) and a slider (520) that slidably mates the core rod (510);
a proximal end of the outer tube body (100) is connected to the core rod (510), and a proximal end of the transmission portion (400) is connected to the slider (520).

12. The surgical instrument according to claim 11, wherein the transmission portion (400) comprises a connecting tube (410) and a cable (420);
the connecting tube (410) slides and fits inside the outer tube body (100), and the connecting tube (410) is connected to the working portion (300); a distal end of cable (420) is connected to connecting tube (410), and a proximal end of cable (420) is connected to slider (520).

13. The surgical instrument according to claim 12, wherein a surface of the cable (420) is coated with a lubricating coating.

14. The surgical instrument according to claim 10, wherein the working portion (300) comprises a snare; the snare has an elastic tendency to expand radially.

15. A medical catheter manufacturing method **characterised in that** the medical catheter manufacturing method comprises following steps:
installing a support tube (200) inside an outer tube body (100);
wherein the support tube (200) has a mesh structure or spiral structure extending along an inner wall of the outer tube body (100);
optionally, the support tube (200) has a fixed portion (201) that is fixed relative to the outer tube body (100), and a movable portion (202) that is movable relative to the fixed portion (201);
the medical catheter manufacturing method further comprises: adjusting the movement of the movable portion (202) relative to the fixed portion (201) to change the mesh size or pitch of the support tube (200).
